# EUROPEAN PATENT SPECIFICATION

(11) **EP 0 998 484 B1**
(45) Date of publication and mention of the grant of the patent: **03.03.2004**
(21) Application number: 98934998.0
(22) Date of filing: 26.06.1998
(51) Int. Cl.: C07J 1/00

(54) **METHOD OF IDENTIFYING COMPOUNDS HAVING REDUCED SYSTEMIC EFFECTS**
VERFAHREN ZUR IDENTIFIZIERUNG VON VERBINDUNGEN MIT VERMINDERTER SYSTEMISCHER AKTIVITÄT
PROCEDE D'IDENTIFICATION DES COMPOSES AYANT UNE ACTIVITE SYSTEMIQUE REDUITE

(30) Priority: 30.06.1997 GB 9713819; 30.06.1997 GB 9713818
(43) Date of publication of application: 10.05.2000
(73) Proprietor: GLAXO GROUP LIMITED, Greenford, Middlesex UB6 0NN (GB)
(72) Inventor: BIGGADIKE, Keith, Stevenage, Hertfordshire SG1 2NY (GB); ANGELL, Richard, Martyn, Stevenage, Hertfordshire SG1 2NY (GB); PROCOPIOU, Panayiotis, Alexandrou, Stevenage, Hertfordshire SG1 2NY (GB); FARRELL, Rosanne Mary, Stevenage, Hertfordshire SG1 2NY (GB); RAMESH, Usha V., Affymax Research Institute, Palo Alto, CA 94304 (US); HOLMES, Duncan Stuart, Stevenage, Hertfordshire SG1 2NY (GB)
(74) Representative: Crawley, Karen Anne
(86) International application number: PCT/EP1998/003905
(87) International publication number: WO 1999/001467

(56) References cited:
- EP-A- 0 200 692
- EP-A- 0 503 388
- WO-A-85/04168
- WO-A-94/08945
- WO-A-96/01322
- WO-A-96/40621
- WO-A-97/24365
- WO-A-97/24367
- WO-A-97/24368
- DE-A- 3 133 631
- GB-A- 2 079 755
- US-A- 4 093 721
- US-A- 4 906 661
- US-A- 4 935 421
- J. E. PIKE ET AL: "Addition of Alkyl Vinyl Ethers to delta-16, 20-Keto Steroids. II." JOURNAL OF ORGANIC CHEMISTRY., vol. 28, no. 10, 11 October 1963, pages 2502-2507, XP002082041 EASTON US
- MORIHIRO MITSUKUCHI ET AL: "STUDIES ON TOPICAL ANTIINFLAMMATORY AGENTS. III. SYNTHESIS OF 17ALPHA-ACYLOCY-9ALPHA-FLUORO-11BETA-HYDRO XY-16BETA-METHYL-1,4- PREGNADIENE-3,20-DIONE 21-THIO DERIVATIVES AND RELATED COMPOUNDS" CHEMICAL AND PHARMACEUTICAL BULLETIN, vol. 37, no. 12, December 1989, pages 3286-3293, XP002026042
- MORIHIRO MITSUKUCHI ET AL: "STUDIES ON TOPICAL ANTIINFLAMMATORY AGENTS. II. SYNTHESIS AND VASOCONSTRICTIVE ACTIVITY OF 21-SUBSTITUTED CORTICOSTEROIDS WITH SULFUR-CONTAINING MOIETIES" CHEMICAL AND PHARMACEUTICAL BULLETIN, vol. 37, no. 7, July 1989, pages 1795-1801, XP002026043
- AL-HABET S M H ET AL: "In vitro hydrolysis of steroid acid ester derivatives of prednisolone in plasma of different species" JOURNAL OF PHARMACEUTICAL SCIENCES., vol. 79, no. 10, October 1990, pages 916-918, XP002095077 WASHINGTON US
- KUMARI D ET AL: "Hydrolysis of methyl steroid-21-oates and acetyl steroid-21-ols by rat liver microsomes" DRUG METAB. DISPOS., vol. 13, no. 5, 1985, pages 627-629, XP002095078
- CHEMICAL ABSTRACTS, vol. 91, no. 19, 5 November 1979 Columbus, Ohio, US; abstract no. 151775, P. PREZIOSI ET AL: "Intrinsic Systemic Activity of Glucocorticoids for Topical Respiratory Use" page 100; column 1; XP002095084 & FARMACO, EDIZIONE PRATICA., vol. 34, no. 8, 1979, pages 325-337, PAVIA IT
- P. PREZIOSI ET AL: "Attivita Intrinseca Sistemica di Glucocorticoidi Ad Uso Topico Respiratorio" FARMACO, EDIZIONE PRATICA., vol. 34, no. 8, 1979, pages 325-337, XP002095079 PAVIA IT
- WEISENBERGER H ET AL: "Species differences in the hydrolysis of dexamethasone 21-isonicotinate by serum esterases" KLIN. WOCHENSCHR. , vol. 50, no. 13, 1972, pages 665-666, XP002095080
- BARROW A ET AL: "Kinetics and disposition of picumeterol in animals" XENOBIOTICA, vol. 25, no. 9, 1995, pages 993-1007, XP002095081
- G. KELSO ET AL: "Apolipoprotein J is Associated with Paraoxonase in Human Plasma" BIOCHEMISTRY, vol. 33, no. 3, 25 January 1994, pages 832-839, XP002095082 EASTON, PA US cited in the application
- M. MACKNESS ET AL: "Substrate Specificity of Human Serum Paraoxonase" BIOCHEMICAL SOCIETY TRANSACTIONS, vol. 19, no. 3, August 1991, page 304S XP002095083

## Description

The present invention relates to the use of pharmaceutical compounds in therapy, particularly in the treatment of conditions of the respiratory tract and conditions of the gastrointestinal tract such as inflammatory and allergic conditions of these and other tissues, while reducing or eliminating undesirable or adverse effects at sites distant from the target tissue. The invention relates also to compounds for use in therapy which have an advantageous side-effect profile, to pharmaceutical formulations thereof and to methods of selecting said compounds.

It is well known that some pharmaceutical compounds useful in therapy may cause, in addition to their desired pharmacological effect, undesirable or adverse side-effects at sites distant from the target tissue, so-called systemic effects. The usefulness of the compound for the treatment of a given disorder depends *inter alia* on the ratio between the potency of the compound in respect of its desired pharmacological activity and its systemic liability.

Glucocorticosteroids (also known as corticosteroids) are one category of known drug widely used for the treatment of inflammatory disorders or diseases such as asthma and rhinitis, which may in general suffer from the disadvantage of causing unwanted systemic effects following administration. Such effects include adrenal suppression, increased bone turnover, impaired growth, skin thinning and easy bruising, and increased risk of cataracts. WO94/13690, WO94/14834, WO92/13873 and WO92/13872 all disclose glucocorticosteroids which are alleged to possess anti-inflammatory activity coupled with reduced systemic potency.

S. Al-Habet et al. J. Pharm. Sci. 79/10, 916(1990) describes the *in vitro* hydrolysis of steroid acid ester derivatives of prednisolone in plasma of different species.

Another class of drug widely used for the treatment of asthma for example are β₂-adrenoreceptor agonists, which also suffer from the disadvantage of causing unwanted systemic effects following administration. Such effects include central nervous system stimulatory effects and cardiac arrhythmia.

US4935421 and US4906661 both describe β-blocking agents that are useful for the treatment of glaucoma. The compounds are described as having a short duration in the systemic circulation, but with good stability in occular fluid and so having a low potential for producing systemic side effects.

One way in which the potential adverse side-effects of a compound may be ameliorated is by seeking to confine the pharmacological activity of the compound to the target tissue or site of action in the body, thereby reducing or eliminating unwanted systemic effects associated with the administration of that compound.

We have now surprisingly found that certain compounds having therapeutic activity are converted in the bloodstream into other compounds which substantially lack said activity. In particular, we have found that certain compounds possessing a 5-membered ring structure which incorporates an ester linkage are hydrolysed rapidly in the blood (plasma) to form compounds which substantially lack said therapeutic activity. Such plasma-labile compounds may thus be expected to have reduced systemic potency compared to compounds which are not plasma-labile.

Whilst not being bound by any theory concerning possible mechanisms of action, it is believed that an enzyme referred to hereinafter as "lactonase" is responsible for the hydrolysis of the aforementioned compounds in the blood.

Thus, we have found a method of localising the therapeutic activity of a compound to a predetermined site within the human or animal body, the method comprising administering a compound or a physiologically acceptable salt or solvate thereof to the desired target tissue of a human or animal subject, said compound having a therapeutic activity and being hydrolysable in the blood to another compound which substantially lacks said therapeutic activity.

We have thus, also found a method of eliciting a therapeutic effect in a target tissue while avoiding concomitant systemic liability, the method comprising administering to a human or animal subject in need of therapy a compound or a physiologically acceptable salt or solvate thereof in an amount sufficient to have therapeutic activity, which compound is hydrolysable in the blood to another compound which substantially lacks said therapeutic activity.

Additionally, we have found a method of treating a disorder with a pharmaceutical compound while reducing or eliminating any systemic effects associated with the administration of that compound, the method comprising administering a therapeutically effective amount of said compound or a physiologically acceptable salt or solvate thereof to a human or animal subject, which compound is hydrolysable in the blood to another compound which substantially lacks said therapeutic activity.

Furthermore, we have found that it is possible to modify the chemical structures of known ('parent') drug compounds in such a manner that the modified compound retains the desired therapeutic activity, but differs from the 'parent' compound in that it is hydrolysable in the blood to a compound which substantially lacks the therapeutic activity of the 'parent' compound. Thus, we have found a method of reducing the systemic effects associated with the administration of a drug compound, the method comprising modifying said compound such that the modified form of the compound retains the desired therapeutic activity and is rendered hydrolysable in the blood to a compound which substantially lacks said desired therapeutic activity.

There is provided a method of identifying a compound capable of providing a therapeutic effect at a target site within a human or animal body with reduced systemic potency to said body comprising
(a) comparing the susceptibility to hydrolysis of said compound in the presence of a purified lactonase enzyme or a recombinant form thereof to the corresponding susceptibility in the absence of said lactonase enzyme; and
(b) selecting a compound on the basis of enhanced susceptibility to hydrolysis in the presence of the lactonase enzyme.

The susceptibility to hydrolysis is preferably compared by means of the 'enzymatic hydrolysis test method' defined herein.

### Compounds

There is provided a therapeutically active compound or a salt or solvate thereof, hydrolysable in human or animal blood to a compound with reduced therapeutic activity. The therapeutically active compound is other than the compounds disclosed in International Patent Applications Nos. WO97/24365, WO97/24367 and WO97/24368.

The therapeutically active compound preferably comprises a ring structure, more preferably a 5-membered ring structure including an ester linkage, wherein said ester linkage is hydrolysable by a lactonase enzyme. Compounds having an ester linkage herein are defined to also include compounds in which the 'ester (i.e. -CO-O-) linkage' is part of a broader linkage, such as a carbonate (i.e. -O-CO-O-) linkage.

The compounds herein are therapeutically active. Preferred are those compounds which are useful for the treatment of respiratory disorders and disorders of the gastrointestinal tract, skin, eyes and joints. Also preferred are anti-inflammatory or anti-allergic compounds such as corticosteroids which have utility in the treatment of *inter alia* allergic and inflammatory conditions of the aforementioned tissues. Also preferred are β₂-adrenoreceptor agonists.

The compounds herein are hydrolysable in human or animal blood to a compound with reduced therapeutic activity. By "therapeutic activity" is meant the pharmacological activity for which the compound is administered. By "a compound with reduced therapeutic activity" it is meant a compound which is less potent in terms of its desired pharmacological activity compared to the parent compound. Preferably, the hydrolysate of the parent compound is at least 2-fold less potent, particularly 5-fold less potent and especially at least 10-fold less potent than the parent compound.

In a preferred aspect, the compounds herein are hydrolysable by a lactonase enzyme.

Suitably, the lactonase enzyme has a molecular weight of approximately 40 kda and is:
- insensitive to phenylmethylsulphonyl fluoride (PMSF) at a concentration of 10 mM (alkylates serine residues in the Ser/His/Asp catalytic triad of classical Ser proteases and esterases);
- insensitive also to p-chloromercuribenzoate (PCMB) at a concentration of 1 mM (alkylates Cys residues in the Cys/His/Asp catalytic triad of classical Cys proteases and esterases);
- insensitive to eserine at a concentration of 50 mM;
- Ca²⁺-dependent. This last dependence is reversible, i.e. EDTA can be used to chelate Ca²⁺ with concomitant loss of activity which can be recovered by addition of Ca²⁺.

Enzymes possessing a similar profile are described in the prior art. For example, W.N. Fishbein *et al*, Journal of Biological Chemistry 1966, 241(21), 4835-4841, describe the purification of a γ-lactonase (i.e. an enzyme capable of hydrolysing aliphatic γ-lactones) from rat liver and human plasma. Further, it is believed that the enzyme "lactonase" is related to or substantially homologous to the enzyme paraoxonase disclosed in International Patent Application No. WO 96/01322 and C. E. Furlong et al. Chem. Biol. Interactions, Vol 87, p35-48, (1993), the contents of both of which are incorporated herein by reference as if reproduced in full below. Paraoxonase is desribed by G.J. Kelso, Biochem. 1994, 33, 832-839 to be present in the liver and the blood, but absent from the lung, heart, brain, placenta, skeletal muscle, kidney and pancreas.

### Enzymatic hydrolysis test method

The compounds herein have relatively short half-lives in blood *in vitro*. A test method for determining the half-life of the compounds under defined enzymatic hydrolysis conditions *in vitro* is now described. The test method is believed to provide a suitable indicator as to effects *in vivo*. In the test method, the hydrolysis of test compounds by a lactonase enzyme is monitored using RP HPLC with UV detection.

The 'enzymatic hydrolysis test method' is as follows: Incubations are carried out in 1ml volumes in an aqueous medium containing 5% bovine serum albumin in the presence of 20mM CaCl₂. The solutions are preincubated at 37°C for 5 minutes before the addition of the test compound (5µl of a 5mg/ml solution in DMSO) and then lactonase enzyme (10µl to the 1ml incubations). Control incubations containing no enzyme are also included. The enzymatic hydrolysis is monitored by removal of aliquots and quenching the reaction by the addition of an equal volume of acetonitrile. The samples are vortex mixed, then centrifuged and the supernatants are transferred to autosampler vials for HPLC analysis.

In a suitable HPLC procedure, aliquots (20µl) of the supernatants are injected onto a Zorbax Rx C8 column (250 x 4.6mm; Hichrom). The column is maintained at 40°C and eluted at a flow rate of 1.0 mL/min with a mobile phase of acetonitrile: 50 mM ammonium formate (65:35) adjusted to pH 4.2 with formic acid. Detection is by UV absorbance at 240nm, and chromatographic peak areas for both parent and metabolite are measured.

In a preferred aspect, the half-life of each compound may be determined by a method in which peak areas are plotted against time on a log-linear scale, and the half lives determined by extrapolation or interpolation of a straight line joining two points.

The above described 'enzymatic hydrolysis test method' employs lactonase enzyme. Suitable forms of lactonase enzyme include human serum paraoxonase or a recombinant form thereof, or purified lactonase, obtained from human plasma as described hereinafter. Purification of human serum paraoxonase is described by C E Furlong et al, Chem.Biol.Interactions, Vol 87, p35-48, (1993) and recombinant human serum paraoxonase is described in International Patent application No. WO 96/01322

Generally, the compounds for use in the invention have a half-life in the presence of lactonase enzyme of less 1 hour, preferably less than 30 minutes, especially less than 10 minutes. Correspondingly, the compounds would also be expected to have a half-life in human plasma of less 1 hour, preferably less than 30 minutes, especially less than 10 minutes (see later described 'stability in human plasma' test method). As a result of this rapid hydrolysis in the presence of lactonase enzyme the compounds are likely to possess reduced systemic potency. Such compounds may thus, represent a safer alternative to plasma-stable drugs which are more likely to have poor side-effect profiles.

### Structure of the compounds

Compounds for use in the invention typically contain a ring structure, preferably a 5-membered ring structure, which incorporates an ester linkage. The ester linkage is susceptible to hydrolysis by lactonase enzyme.

### Lactone-like compounds

Preferred compounds include those containing a lactone-like group, preferably a lactone group, most preferably a γ-lactone group. In the case of such a compound being a steroid derivative, the lactone-like group may be either fused to a ring of the steroid nucleus or connected to the steroid nucleus via an appropriate linker group. Preferably the lactone-like group is fused or connected to the cyclopentane ring (conventionally known as ring D) of the steroid nucleus.

Ilustrative lactone-like compounds include:
6α,9α-Diffuoro-11β-hydroxy-16α-methyl-17-spiro[androsta-1,4-diene-17,5'-[1,3]oxathiolane]-2',3,4'-trione;
6α,9α-Difluoro-11β-hydroxy-16α-methyl-3-oxo-17α-propionyloxy-androsta-1,4-diene-17β-carbothioic acid *S*-(2-oxo-tetrahydro-furan-3-ylmethyl) ester;
6α,9α-Difluoro-11β-hydroxy-16α-methyl-3-oxo-17α-(2-oxo-tetrahydrofuran-4-ylsulfanyl-acetoxy)-androsta-1,4-diene-17β-carbothioic acid methyl ester;
6α,9α-Difluoro-11β,21-dihydroxy-16α,17α-[2-(2-oxo-tetrahydrofuran-3-yl)sulfanyl]ethylidenedioxy-pregn-4-ene-3,20-dione;
9α-Fluoro-11β,17α,21-trihydroxy-3,20-dioxo-pregna-1,4-diene-16α-acetic acid γ-lactone;
3-[3-[2-(4-Amino-3,5-dichlorophenyt)-2-hydroxyethylamino]propylsulfanyl]-dihydro-furan-2-one trifluoroacetate;
and salts and solvates thereof.

It will be appreciated that some of the above described lactone-like compounds for use in the invention have individual R and S diastereoisomeric forms at the asymmetric centre at the point of attachment of the lactone-like 5-membered ring; these individual isomers are included within the scope of the invention as well as the mixtures thereof. It will further be appreciated that the compounds for use in the invention may include the individual R and S diastereoisomers at other asymmetric centres. Thus, individual R and S diastereoisomers isolated such as to be substantially free of the other diastereoisomer, i.e. pure, and mixtures thereof are included within the scope of the present invention. An individual R or S diastereoisomer isolated such as to be substantially free of the other diastereoisomer, i.e. pure, will preferably be isolated such that less than 10%, preferably less than 1%, especially less than 0.1%, of the other diastereoisomer is present.

### Cyclic carbonate compounds

Other suitable compounds include a ring structure, preferably a 5-membered ring structure, having a carbonate (i.e. -O-CO-O-) linkage. Preferred compounds of this type include those of formula (Ia) or (Ib) 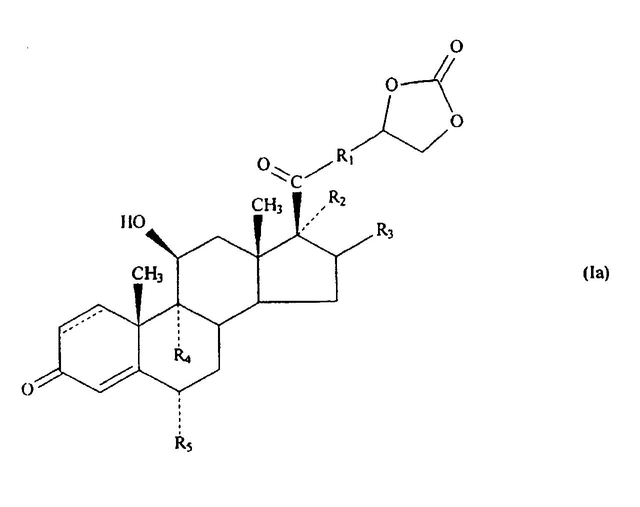 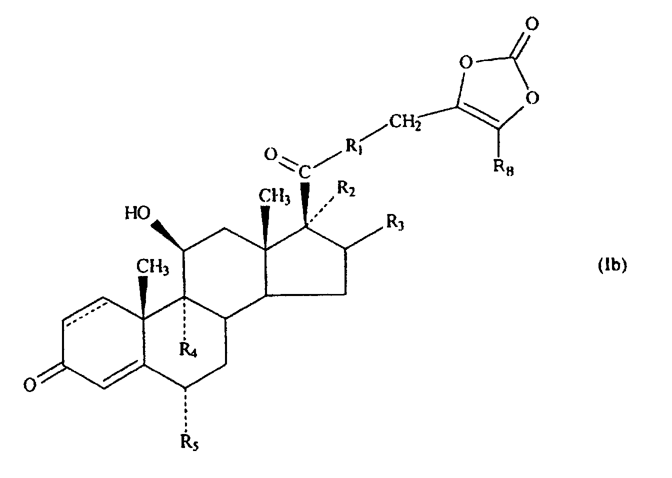 and solvates thereof, in which
R₁ represents O or S;
R₂ individually represents OC(=O)C₁₋₆ alkyl;
R₃ individually represents hydrogen, methyl (which may be in either the α or p configuration) or methylene;
or R₂ and R₃ together represent 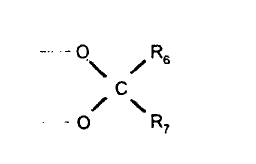 wherein R₆ and R₇ are the same or different and each represents hydrogen or C₁₋₆ alkyl;
R₄ and R₅ are the same or different and each represents hydrogen or halogen;
R₈ represents hydrogen, C₁₋₆ alkyl or aryl; and
― represents a single or a double bond.

In the above definitions, the term "alkyl" as a group or part of a group means a straight chain, or, where available, a branched chain alkyl moiety. For example, it may represent a C₁₋₄ alkyl function as represented by methyl, ethyl, n-propyl, i-propyl, n-butyl and t-butyl.

The solvates may, for example, be hydrates.

References hereinafter to "compounds of formula (I)" include compounds of formula (Ia) and formula (Ib) and all stereoisomers and mixtures thereof.

Diastereoisomers and mixtures thereof at the asymmetric centre formed when R₂ and R₃ together represent 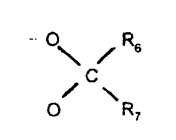 and R₆ and R₇ are different are also included within the scope of the present invention.

Preferably, R₈ represents hydrogen, or methyl.

Preferred are compounds of formula (I) in which R₁ represents S.

Also preferred are compounds of formula (I) in which R₂ individually represents OC(=O)C₁₋₆ alkyl, more preferably OC(=O)C₁₋₃ alkyl, especially OC(=O)ethyl. Compounds within this group in which R₃ is methyl are generally preferred.

Also preferred are compounds of formula (I) in which R₂ and R₃ together represent 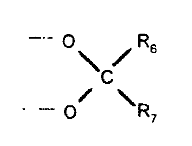 wherein R₆ and R₇ are the same or different and each represents hydrogen or C₁₋₆ alkyl, particularly hydrogen or C₁₋₃ alkyl, especially hydrogen, methyl or n-propyl.

Compounds of formula (I) in which R₄ and R₅, which can be the same or different, each represents hydrogen, fluorine or chlorine, particularly hydrogen or fluorine, are preferred. Especially preferred are compounds in which both R₄ and R₅ are fluorine.

Particularly preferred are compounds of formula (I) in which R₁ is S; R₂ is OC(=O)C₁₋₆ alkyl, particularly OC(=O)C₁₋₃ alkyl, especially OC(=O)ethyl; R₃ is methyl; and R₄ and R₅, which can be the same or different, each represents hydrogen or fluorine, especially fluorine.

Also particularly preferred are compounds of formula (I) in which R, is S; R₂ and R₃ together represent 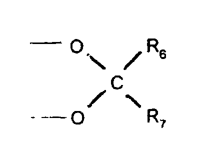 wherein R₆ and R₇ are the same or different and each represents hydrogen or C₁₋₆ alkyl, particularly hydrogen or C₁₋₃ alkyl, especially hydrogen, methyl or n-propyl; and R₄ and R₅ which can be the same or different each represents hydrogen or fluorine, especially fluorine. The R-isomers of compounds within this group in which R₆ and R₇ are different are preferred.

It will be appreciated that each of the above compounds of formula (Ia) includes the individual R and S diastereoisomers at the asymmetric centre at the point of attachment of the cyclic carbonate ring as well as the mixtures thereof. It will further be appreciated that the compounds of formula (I) may include the individual R and S diastereoisomers at the asymmetric centre formed when R₂ and R₃ together represent 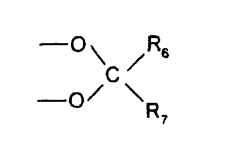 wherein R₆ and R₇ are different, as well as mixtures thereof.

Preferred compounds of formula (I) include:
6α,9α-Diftuoro-11β-hydroxy-16α-methyl-3-oxo-17α-propionyloxy-androsta-1,4-diene-17β-carbothioic acid *S*-(2-oxo-1,3-dioxolan-4-yl) ester;
6α,9α-Difluoro-11β-hydroxy-16α,17α-isopropylidenedioxy-3-oxo-androsta-1,4-diene-17β-carbothioic acid *S*-(2-oxo-1,3-dioxolan-4-yl) ester;
6α,9α-Difluoro-11β-hydroxy-16α-methyl-3-oxo-17α-propionyloxy-androsta-1,4-diene-17β-carboxylic acid *O*-(2-oxo-1,3-dioxolan-4-yl) ester;
6α,9α-Difluoro-11β-hydroxy-16α-methyl-3-oxo-17α-propionyloxy-androsta-1,4-diene-17β-carbothioic acid *S*-(5-methyl-2-oxo-1,3-dioxol-4-ylmethyl) ester;
6α,9α-Difluoro-11β-hydroxy-16α,17α-isopropylidenedioxy-3-oxo-androsta-1,4-diene-17β-carbothioic acid *S*-(5-methyl-2-oxo-1,3-dioxol-4-ylmethyl) ester;
6α,9α-Difluoro-11β-hydroxy-16α-methyl-3-oxo-17α-propionyloxy-androsta-1,4-diene-17β-carboxylic acid *O*-(5-methyl-2-oxo-1,3-dioxol-4-ylmethyl) ester;
and solvates thereof.

The compounds of formula (I) and solvates thereof may be prepared by the methodology described hereinafter.

According to a first process (A), a compound of formula (Ib) may be prepared by treating a compound of formula (II) 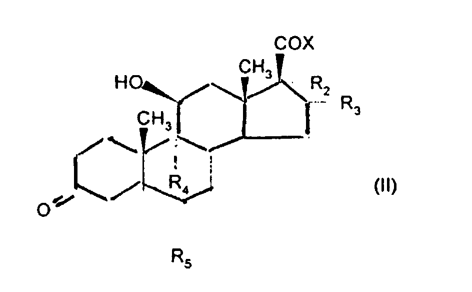 in which R₂, R₃, R₄, R₅ and ― are as defined hereinbefore for compounds of formula (I) and X represents OH or an activated derivative thereof such as a triazole or a mixed anhydride, with a compound of formula (III) 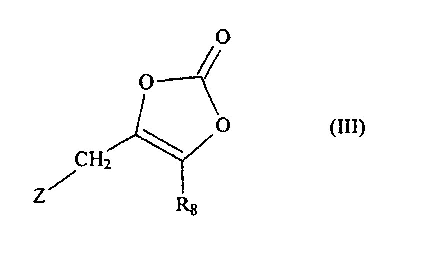 and salts thereof, in which
Z represents OH or SH, and R₈ is as defined hereinbefore for compounds of formula (Ib).

Thus, a compound of formula (II) wherein X represents OH may be activated with an activating agent such as a triazole e.g. 1-hydroxy-benzotriazole and a carbodiimide such as 1-(3-dimethylamino-propyl)-3-ethyl-carbodiimide hydrochloride in a polar solvent such as dimethylformamide, conveniently at elevated temperatures e.g. about 100°C, and under an inert atmosphere such as nitrogen or the like, to form an activated derivative of the compound of formula (II), such as a triazole derivative e.g. a benzotriazole derivative of formula (IV) 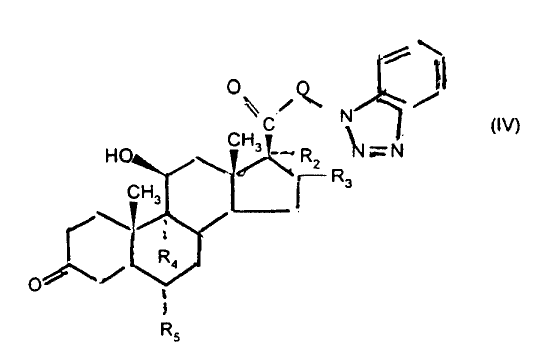 (in which R₂, R₃, R₄, R₅ and ― are as defined hereinbefore).

The activated derivative, which may be isolated if required, is reacted with a compound of formula (III) as defined above to form the desired compound of formula (Ib).

It will be appreciated by those skilled in the art that the coupling reaction may take place in one step without the isolation of the activated derivative if a compound of formula (III) is present during or added following activation. Alternatively, the activated derivative may be isolated and then subsequently treated with a compound of formula (III) to form the desired compound of formula (Ib).

Compounds of formula (Ib) may also be prepared according to the above process (A) by coupling a compound of formula (II) wherein X represents OH with a compound of formula (III) as defined above *via* an intermediate mixed anhydride, for example, a mixed phosphate anhydride such as a compound of formula (V) as described by Kertesz and Marx in the Journal of Organic Chemistry, 1986, 51, 2315-2328.

Thus, a compound of formula (II) wherein X represents OH may be activated with an activating agent, such as diethylchlorophosphate in the presence of a base such as a tertiary amine e.g. triethylamine and in a suitable solvent such as a chlorinated solvent e.g. dichloromethane to form an activated derivative of the compound of formula (II) e.g. a diethylphosphate mixed anhydride derivative of formula (V) 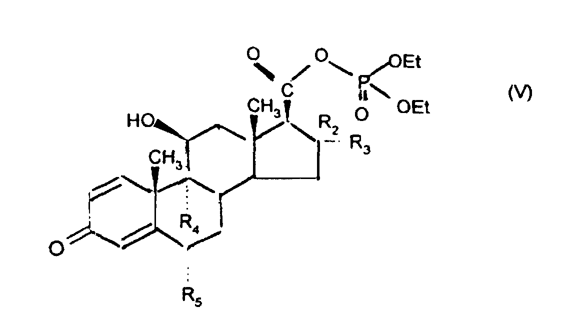 (in which R₂, R₃, R₄, R₅ and ― are as defined hereinbefore).

The activated derivative, which may be isolated if required, is reacted with a compound of formula (III) as defined above to form the desired compound of formula (Ib).

It will be appreciated by those skilled in the art that the coupling reaction may take place without the isolation of the activated derivative if a compound of formula (III) is present during or added following activation. Alternatively, the activated derivative may be isolated and then subsequently treated with a compound of formula (III) to form the desired compound of formula (Ib).

Compounds of formula (I) wherein R₁ represents O or S may also be prepared according to a second process (B) in which a compound of formula (II) in which R₂, R₃, R₄, R₅ and ― are as defined hereinbefore and X represents OH or SH or their corresponding salts, is treated with a compound of formula (VI) or formula (VII) 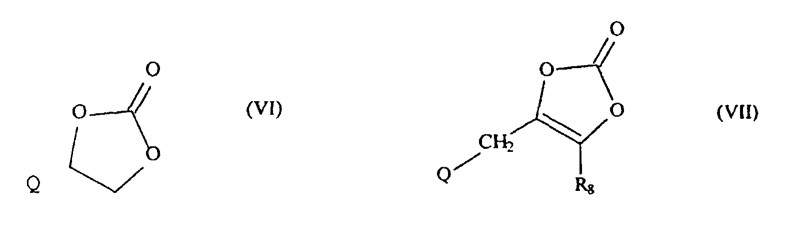 in which Q represents a suitable leaving group (such as Cl, Br, OSO₂A wherein A is, for example CH₃, CF₃, p-CH₃C₆H₄) and R₈ is as defined above, under standard methods.

Compounds of formula (I) wherein R₁ represents O or S may be prepared according to the above process (B) by alkylation of a compound of formula (II) wherein X represents OH or SH respectively, with a compound of formula (VI) or formula (VII) wherein Q represents a suitable leaving group using methods known in the art, or an adaptation of those methods.

Thus, for example, a compound of formula (I) wherein R, represents O may be prepared by alkylation of a compound of formula (II) wherein X represents OH conveniently in the form of an appropriate salt (such as alkali metal e.g. sodium or quarternaryammonium salt) with a compound of formula (VI) or formula (VII) wherein Q represents a suitable leaving group, preferably chlorine, bromine or mesylate. The alkylation reaction is preferably carried out in the presence of a solvent, suitably a polar solvent, under inert conditions, for example, nitrogen or the like, conveniently at a temperature of between about 0°C to 100°C. Suitable polar solvents may include acetone, dimethylformamide, dimethyl acetamide, dimethylsulphoxide, dichloro-methane or chloroform. Preferably, the alkylation reaction is carried out in the presence of a base such as potassium carbonate in an inert solvent such as dimethylformamide, and at a temperature of 0 to 20°C.

Similarly, compounds of formula (I) wherein R₁ represents S can be prepared according to the above process (B) by alkylation of a compound of formula (II) wherein X represents SH with a compound of formula (VI) or formula (VII) wherein Q represents a suitable leaving group by adaptation of the methods described by Phillipps et al, Journal of Medicinal Chemistry, 1994, 37, 3717-3729. Thus, a compound of formula (I) wherein R₁ represents S may be prepared by alkylation of the corresponding compound of formula (II) wherein X represents SH conveniently in the form of an appropriate salt (such as alkali metal e.g. sodium or quarternaryammonium salt) with a compound of formula (VI) or formula (VII) wherein Q represents a suitable leaving group as described hereinabove for similar alkylation reactions.

Alternatively, compounds of formula (Ib) wherein R₁ represents O or S may be prepared according to the above process (B) by alkylation of a compound of formula (II) wherein X represents OH or SH with a compound of formula (VII) wherein Q represents OH under Mitsunobu conditions using triphenylphosphine and a dialkyl azodicarboxylate, or by using Vilsmeier methodology as described by Barrett and Procopiou in the Journal of the Chemical Society, Chemical Communications, 1995, 1403-1404.

Compounds of formula (I) may also be prepared from other compounds of formula (I) thereof using conventional interconversion procedures such as transacetalisation or epimerisation. Thus, a process for preparing a compound of formula (I) by interconversion of another compound of formula (I) (process C) constitutes a further aspect of the present invention.

Compounds of formula (I) having a 1,2 single bond may be prepared by partial reduction of the corresponding 1,2 double bond compound by conventional methods. Thus, for example, by hydrogenation of the corresponding compound of formula (I) or of an intermediate used for the preparation of a compound of formula (I) using a palladium catalyst, conveniently in a suitable solvent e.g. ethyl acetate or preferably by using tris(triphenylphosphine) rhodium (I) chloride (known as Wilkinson's catalyst), conveniently in a suitable solvent such as toluene, ethyl acetate or ethanol.

It will be appreciated by those skilled in the art that it may be desirable to use protected derivatives of intermediates used in the preparation of compounds of formula (I). Thus, the above processes may require deprotection as an intermediate or final step to yield the desired compound. Thus, according to another process (D), a compound of formula (I) may be prepared by subjecting a protected derivative of a compound of formula (I) to reaction to remove the protecting group or groups present, constituting a further aspect of the present invention.

Protection and deprotection of functional groups may be effected using conventional means. Thus, hydroxyl groups may be protected using any conventional hydroxyl protecting group, for example, as described in Protective Groups in Organic Chemistry, Ed. J.F.W. McOmie (Plenum Press, 1973) or Protective Groups in Organic Synthesis by Theodora W. Green (John Wiley and Sons, 1991).

Examples of suitable hydroxyl protecting groups includes groups selected from alkyl (e.g. t-butyl or methoxymethyl), aralkyl (e.g. benzyl, diphenylmethyl or triphenylmethyl), heterocyclic groups such as tetrahydropyranyl, acyl (e.g. acetyl or benzoyl) and silyl groups such as trialkylsilyl (e.g. t-butyldimethylsilyl). The hydroxyl protecting groups may be removed by conventional techniques. Thus, for example alkyl, silyl, acyl and heterocyclic groups may be removed by solvolysis, e.g. by hydrolysis under-acidic or basic conditions. Aralkyl groups such as triphenylmethyl may be similarly be removed by solvolysis, e.g. by hydrolysis under acidic conditions. Aralkyl groups such as benzyl may be cleaved by hydrogenolysis in the presence of a Noble metal catalyst such as palladium-on-charcoal.

The compounds of formulae (II), (III), (IV), (V), (VI) and (VII) are either generally known compounds or may be prepared by methods analogous to those described in the art for preparing the known compounds of formula (II), (III), (IV), (V), (VI) and (VII) or may be prepared by the methods described herein. Novel compounds of formulas (II), (III), (IV), (V), (VI) and (VII) form a yet further aspect of the present invention.

For example, the compounds of formula (II) wherein X represents OH can be prepared by oxidation of an appropriate 21-hydroxy-20-keto-pregnane of formula (VIII) 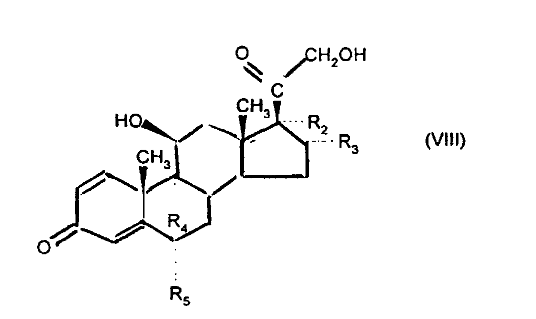 (in which R₂, R₃, R₄, R₅ and ― are as defined hereinbefore) using, for example, the methodology described by Kertesz and Marx, Journal of Organic Chemistry, 1986, 51, 2315-2328.

Compounds of formula (VIII) are commercially available, for example, fluocinolone acetonide, budesonide and triamcinolone acetonide are available from Sigma-Aldrich, or can be prepared from the commercially available compounds of formula (VIII) by, for example, the transacetalisation methods described in EP0262108 and by partial reduction of the 1,2 double bond compounds by the methods described herein. Alternatively, compounds of formula (VIII) can be prepared from commercially available 17α-hydroxyl derivatives of compounds of formula (VIII), for example, betamethasone, flumethasone, prednisolone, beclomethasone, and dexamethasone available from Sigma-Aldrich, by esterification of the 17α-hydroxyl group according to the method described by Gardi et al, Tetrahedron Letters, 1961, 448. Novel compounds of formula (VIII) form yet a further aspect of the present invention.

Compounds of formula (II) wherein X represents SH can be prepared by the application or adaptation of known methods, for example, using methods described by Phillipps et al, Journal of Medicinal Chemistry, 1994, 37, 3717-3729.

Compounds of formula (III), (VI) and (VII) are commercially available from Sigma-Aldrich or may be readily prepared by application or adaptation of known methods. For example, compounds of formula (III) wherein Z is OH and R₈ is methyl can be prepared by the method of Miyauchi et al, Chem. Pharm. Bull. 1990, 38, 1077-1078; compounds of formula (III) wherein Z is OH and R₈ is hydrogen can be prepared by the method of Jung et al, Heterocycles 1989, 28, 93-97; compounds of formula (III) wherein Z is SH can be prepared from the corresponding compounds wherein Z is bromine, by displacing the bromine atom using e.g. potassium thioacetate, and then hydrolysing the product in a conventional manner; compounds of formula (VII) wherein Q is bromine and R₈ is hydrogen can be prepared by the method of Wender et al, Tetrahedron Letters 1990, 31, 6605-6608; and the compound of formula (VII) wherein Q is bromine and R₈ is methyl by the methods described in W.S. Saari et al., J. Med. Chem. 1984, 27, 713.

Individual isomers of formula (Ia) at the point of attachment of the cyclic carbonate ring moiety may either be prepared from starting materials having the desired stereochemistry or by epimerisation, resolution, fractional crystallisation or chromatography (e.g. HPLC separation) at an appropriate stage in the synthesis of the required compounds of formula (Ia) using conventional means.

Thus, for example, it will be appreciated that synthesis employing a racemic mixture of compounds of formula (VI) will afford compounds of formula (Ia) as a mixture of diastereoisomers, which may then be separated. Alternatively, the individual diastereoisomers may be prepared by employing compounds of formula (VI) in enantiomerically pure form.

Similarly, compounds of formula (I) in which R₂ and R₃ together represent 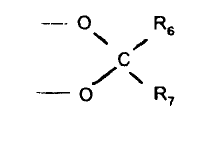 wherein R₆ and R₇ are different, may exist in the R and S diastereoisomeric forms. Synthesis of such compounds may be stereospecific to yield individual diastereoisomers. Thus, for example, the R-diastereoisomer of a compound of formula (I) wherein R₆ represents H and R₇ represents n-propyl may be conveniently prepared by transacetalisation of the corresponding 16α,17α-isopropylidenedioxy derivative with butyraldehyde in the presence of an acid catalyst, such as perchloric acid, as described in EP0262108. The transacetalisation reaction may be performed at an intermediate stage or after introduction of the lactone group.

Solvates (e.g. hydrates) of a compound of formula (I) may be formed during work-up procedure of one of the aforementioned process steps. Thus, the compounds of formula (I) may be isolated in association with solvent molecules by crystallisation from or evaporation of an appropriate solvent to give the corresponding solvates.

### Methods of medical treatment

As mentioned above, the compounds for use in the invention have utility in the treatment of a wide variety of diseases and conditions in human or veterinary medicine. The compounds for use in the invention have particular utility as anti-inflammatory and anti-allergic agents, especially for the treatment of disorders of the respiratory and gastrointestinal tracts.

Examples of disease states in which the compounds for use in the invention have utility include skin diseases such as eczema, psoriasis, allergic dermatitis, neurodermatitis, pruritis and hypersensitivity reactions; inflammatory conditions of the nose, throat or lungs such as asthma (including allergen-induced asthmatic reactions), rhinitis (including hayfever), nasal polyps, chronic obstructive pulmonary disease, interstitial lung disease, and fibrosis; auto-immune diseases such as rheumatoid arthritis; and inflammatory conditions of the gastrointestinal tract such as urticaria and inflammatory bowel conditions such as ulcerative colitis and Crohn's disease. Compounds for use in the invention may also have utility in the treatment of disorders of the eye, such as conjunctiva and conjunctivitis.

It will be appreciated by those skilled in the art that reference herein to treatment extends to prophylaxis as well as the treatment of established conditions.

There is thus provided as a further aspect of the invention a compound as hereinbefore defined or a physiologically acceptable salt or solvate thereof for use in human or veterinary therapy, particularly topical or local therapy, more particularly in the treatment of patients with inflammatory and/or allergic conditions, especially with respiratory disorders or disorders of the gastrointestinal tract.

According to another aspect of the invention, there is provided the use of a compound as hereinbefore defined or a physiologically acceptable salt or solvate thereof for the manufacture of a medicament for use in therapy, particularly topical or local therapy, particularly for the treatment of patients with inflammatory and/or allergic conditions, especially with respiratory disorders or disorders of the gastrointestinal tract.

In a particularly preferred aspect there is provided a method of providing localised therapeutic effect at a target site within a human or animal body. By target site it is meant the site at which the therapeutic effect is desired. Examples of suitable targets would include the lung, where therapeutic respiratory effect is desired, or the gastrointestinal tract, where therapeutic gastronintestinal effect is desired. The method comprises administering a compound to the target site. The compound is generally administered in "a therapeutically effective amount", that is to say an amount sufficient to alleviate the condition or disorder for which the compound is administered. The compound is hydrolysable in human or animal blood to a compound with reduced therapeutic activity.

### Assay or screening method

According to a preferred aspect of the present invention there is provided an assay or screening method for identifying a compound capable of providing a therapeutic effect at a target site within a human or animal body with reduced systemic potency to said body.

The method relies on a comparison of the susceptibility to hydrolysis of the compound in the presence of lactonase enzyme with that of the corresponding susceptibility in the absence of said lactonase enzyme. A compound is selected if it has enhanced susceptibility to hydrolysis in the presence of the lactonase enzyme. The susceptibility to hydrolysis is preferably compared by means of the 'enzymatic hydrolysis test method' defined herein.

The lactonase enzyme is preferably human serum paraoxonase or a recombinant form thereof, or purfied lactonase enzyme obtained from plasma. Preferred compounds have a half-life in the presence of lactonase enzyme of less than 1 hour, preferably less than 30 minutes, more preferably less than 10 minutes.

### Pharmaceutical preparations

The compounds of the invention may be formulated for administration in any convenient way, and the invention therefore also includes within its scope pharmaceutical compositions comprising a compound as hereinbefore defined or a physiologically acceptable salt or solvate thereof in admixture with one or more physiologically acceptable diluents or carriers.

Further, there is provided a process for the preparation of such pharmaceutical compositions which comprises mixing the ingredients. The compounds of use in the invention may, for example, be formulated for oral, buccal, sublingual, local or rectal administration, especially local administration.

Local administration as used herein, includes administration by insufflation and inhalation. Examples of various types of preparation for local administration include ointments, lotions, creams, gels, foams, preparations for delivery by transdermal patches, powders, sprays, aerosols, capsules or cartridges for use in an inhaler or insufflator or drops (e.g. eye or nose drops), solutions/suspensions for nebulisation, suppositories, pessaries, retention enemas and chewable or suckable tablets or pellets (e.g. for the treatment of aphthous ulcers) or liposome or microencapsulation preparations.

Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents, suspending agents or preservatives.

Spray compositions may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain a compound of the invention and a suitable propellant such as a fluorocarbon or hydrogen-containing chlorofluorocarbon or mixtures thereof, particularly hydrofluoroalkanes, especially 1,1,1,2-tetrafluoroethane, 1,1,1,2,3,3,3-heptafluoro-n-propane or a mixture thereof. The aerosol composition may optionally contain additional formulation excipients well known in the art such as surfactants e.g. oleic acid or lecithin and cosolvents e.g. ethanol.

According to a further aspect of the invention, there is provided a pharmaceutical aerosol formulation comprising a compound as hereinbefore defined or a physiologically acceptable salt or solvate thereof, and a fluorocarbon or hydrogen-containing chlorofluorocarbon as propellant, optionally in combination with a surfactant and/or a cosolvent.

Advantageously, the formulations of the invention may be buffered by the addition of suitable buffering agents.

Capsules and cartridges for use in an inhaler or insufflator, of for example gelatine, may be formulated containing a powder mix for inhalation of a compound for use in the invention and a suitable powder base such as lactose or starch. Each capsule or cartridge may generally contain between 20µg-10mg of the compound for use in the invention. Alternatively, the compound for use in the invention may be presented without excipients such as lactose.

The proportion of the active compound for use in the invention in the local compositions according to the invention depends on the precise type of formulation to be prepared but will generally be within the range of from 0.001 to 10% by weight. Generally, however for most types of preparations advantageously the proportion used will be within the range of from 0.005 to 1% and preferably 0.01 to 0.5%. However, in powders for inhalation or insufflation the proportion used will be within the range of from 0.1 to 5%.

Aerosol formulations are preferably arranged so that each metered dose or "puff' of aerosol contains 20µg-2000µg, preferably about 20µg-500µg of a compound for use in the invention. Administration may be once daily or several times daily, for example 2, 3, 4 or 8 times, giving for example 1, 2 or 3 doses each time. The overall daily dose with an aerosol will be within the range 100µg-10mg preferably, 200µg-2000µg. The overall daily dose and the metered dose delivered by capsules and cartridges in an inhaler or insufflator will generally be double those with aerosol formulations.

For internal administration the compounds according to the invention may, for example, be formulated in conventional manner for oral or rectal administration. Formulations for oral administration include syrups, elixirs, powders, granules, tablets and capsules which typically contain conventional excipients such as binding agents, fillers, lubricants, disintegrants, wetting agents, suspending agents, emulsifying agents, preservatives, buffer salts, flavouring, colouring and/or sweetening agents as appropriate. Dosage unit forms are, however, preferred as described below.

Preferred forms of preparation for internal administration are dosage unit forms i.e. tablets and capsules. Such dosage unit forms contain from 0.1mg to 20mg preferably from 2.5 to 10mg of the compounds for use in the invention.

In general terms preparations, for internal administration may contain from 0.05 to 10% of the active ingredient dependent upon the type of preparation involved. The daily dose may vary from 0.1mg to 60mg, e.g. 5-30mg, dependent on the condition being treated, and the duration of treatment desired.

Slow release or enteric coated formulations may be advantageous, particularly for the treatment of inflammatory bowel disorders and inflammatory disorders of the gastrointestinal tract.

The pharmaceutical compositions according to the invention may also be used in combination with another therapeutically active agent. For example, when the compound of the invention is a steroid, this could be used in combination with a β₂-adrenoreceptor agonist, an anti-histamine or an anti-allergic, especially a β₂-adrenoreceptor agonist. The invention thus provides, in a further aspect, a combination comprising a compound of the invention or a physiologically acceptable salt or solvate thereof together with another therapeutically active agent.

The combination referred to above may conveniently be presented for use in the form of a pharmaceutical formulation and thus pharmaceutical formulations comprising a combination as defined above together with a pharmaceutically acceptable diluent or carrier represent a further aspect of the invention.

The individual compounds of such combinations may be administered either sequentially or simultaneously in separate or combined pharmaceutical formulations. Appropriate doses of known therapeutic agents will be readily appreciated by those skilled in the art.

The following Examples illustrate the invention but do not limit the invention in any way.

### EXAMPLES

### A. Purification of Enzyme from human plasma

The basic method steps used to isolate and purify lactonase enzyme from human plasma may be summarised as follows:
- the blood is centrifuged and the plasma retained;
- the plasma is passed through an affinity chromatography column such as a column of Cibracon Blue matrix to remove albumin from the plasma, then through an ion exchange column containing an anion exchange matrix such as quaternary ammonium anion exchange matrix to separate and purify the plasma proteins;
- the enzyme activity is recovered, precipitated using ammonium sulphate and then further purified using a hydrophobic interaction chromatography (HIC) column, a Gel Permeation Chromatography (GPC) matrix, and finally heparin/lectin affinity matrices.

The following detailed purification procedure was employed for this example:
Human blood (600ml) from several volunteers was pooled in heparinised tubes (250 units heparin/ml blood) and spun at 3501g using a Heraeus Labofuge 400R centrifuge at 4°C. Plasma (300ml) was decanted, pooled and stored at -20°C until required. After thawing overnight at 4°C, the plasma was diluted with 5 times its own volume of a loading buffer A (25 mM HEPES, 6 mM calcium chloride, 5 mM dithiothreitol, pH 6.95) and centrifuged for 20 minutes in 20 ml aliquots to remove particulate matter. The diluted plasma was then loaded onto a pseudo affinity chromatography column containing Cibracon Blue matrix, to selectively remove albumin from the plasma. This column was coupled in series to an ion exchange column containing Q-Sepharose matrix (quaternary ammonium anion exchange matrix). The two columns were washed with loading buffer A until the UV absorbance (at 280nm) of the eluting solution reached baseline value. The Cibracon Blue column was removed and the Q-Sepharose column washed in turn with sodium chloride solution in loading buffer A, then sodium chloride solution to ensure that the column did not contain further bound protein. Finally, the column was washed with 1.0M sodium chloride. Fractions containing the lactonase activity were pooled and solid ammonium sulphate (up to 4.1M) was slowly added to precipitate most of the soluble proteins. This was then recovered by centrifugation at 3501g for 30 minutes at 20°C. The pellet fraction which contained lactonase activity was dissolved in 1.64 M ammonium sulphate buffer and then loaded onto a hydrophobic interaction chromatography (HIC) column. Lactonase activity appeared to bind to the matrix. The column was washed with an ammonium sulphate solution and then further washed with buffer until the UV absorbance reached baseline value. The pooled active fractions were diluted with buffer and loaded onto the next column, which contained ceramic hydroxylapatite (Biorad). The unbound fraction did not contain lactonase activity. The column was washed with a potassium phosphate solution in a loading buffer. Protein eluting was found to contain the lactonase activity and these fractions were therefore pooled and concentrated using ultrafiltration concentrator units (Amicon). All of the concentrated pooled active fraction was loaded onto a column containing a Gel Permeation Chromatography matrix, Superdex 200 (Pharmacia). The eluted fractions containing the lactonase activity were pooled and stored at -20°C. The pooled active fraction was concentrated to <0.5ml using ultrafiltration concentrator units (Amicon). All of the concentrated pooled active fraction was loaded onto the final chromatographic step which used two columns in series. The first was a column containing heparin affinity matrix and the second column contained wheat germ lectin affinity matrix.
Lactonase activity was observed in the void volume, showing that it does not bind to heparin or wheat germ lectin under the conditions described.

### B. Biochemical characterisation of the purified enzyme

The enzyme activity purified according to A. above was tested with classical inhibitors in order to establish to which enzyme family it belongs. Also, it was investigated whether a divalent cation was required as a cofactor, as disclosed in W.N. Fishbein *et al*, Journal of Biological Chemistry 1966, 241(21), 4835-4841.

The "lactonase" activity investigated was not inhibited by phenylmethylsulphonyl fluoride (PMSF) (10mM) or eserine (50mM), slightly inhibited by *p*-chloromercuribenzoate (PCMB) (1mM) and totally inhibited by zinc sulphate (1mM), EDTA (1mM) and EGTA (1mM). The activity in the presence of EDTA and EGTA is fully restored if CaCl₂ (100mM) is added. This activity is not restored with MgCl₂ (100mM). These data appear to suggest that "lactonase": (i) may differ from known "classical" aryl esterases in its insensitivity to PCMB; (ii) may not be a carboxyl esterase due to its lack of sensitivity to PMSF; and (iii) is unlikely to be a cholinesterase since it was not inhibited by eserine. The calcium dependence data suggest that the "lactonase" enzyme is likely to be related to that reported by W.N. Fishbein *et al*, Journal of Biological Chemistry 1966, 241(21), 4835-4841.

The molecular weight of the "lactonase" activity was investigated using an SDS-PAGE gel electrophoresis technique. One of the visible bands was identified as human serum paraoxonase, as discussed in WO 96/01322. This known enzyme has a molecular weight of about 40 kda.

### C. Compounds

### General

Melting points were determined on a Kofler block and are uncorrected. ¹H-nmr spectra were recorded at 250 or 400 MHz and the chemical shifts are expressed in ppm relative to tetramethylsilane. The following abbreviations are used to describe the multiplicities of the signals: s (singlet), d (doublet), t (triplet), q (quartet), m (multiplet), dd (doublet of doublets), dt (doublet of triplets) and b (broad). MS(TSP+ve) and MS(ES+ve) refer to mass spectra run in positive mode using thermospray or electrospray techniques respectively. HRMS (ES+ve) refers to high resolution electrospray mass spectrum run in positive mode. TLC (thin layer chromatography) was performed on Merck Kieselgel 60 F₂₅₄ plates and column chromatography was performed on Merck Kieselgel 60 (Art. 7734 or 9385). PLC (preparative layer chromatography) was performed on Whatman silica plates. Preparative HPLC (high performance liquid chromatography) was performed on a Gilson Medical Electronics system using the stationary phase indicated in the example. DMF is used as an abbreviation for anhydrous N,N-dimethylformamide. Organic solutions were dried over anhydrous magnesium sulfate.

Where mixtures of isomers resulting from the asymmetric centre in the lactone group have been prepared, these isomers may be separated by conventional chromatography on silica and assigned as isomers A and B respectively in order of elution from the column.

The starting materials and intermediates indicated may be prepared either by methods already known in the art, by the processes described in the literature references given, or according to the description given hereinafter.

### Example 1

### 6α,9α-Difluoro-11β-hydroxy-16α-methyl-17-spiro[androsta-1,4-diene-17,5'-[1,3]oxathiolane]-2',3,4'-trione

1,1'-Carbonyl-diimidazole (1 g, 6.17 mmol) was added in one portion to a stirred solution of 6α,9α-difluoro-11β,17α-dihydroxy-16α-methyl-3-oxo-androsta-1,4-diene-17β-carbothioic acid (1.2 g, 2.91 mmol) in dry DMF (15 ml). [6α,9α-Difluoro-11β,17α-dihydroxy-16α-methyl-3-oxo-androsta-1,4-diene-17β-carbothioic acid is a known material which may be prepared for example according to the method described in GB-A-2088877.] The mixture was stirred under nitrogen for 24 h at room temperature. The solution was partitioned between water (100 ml) and ethyl acetate (100 ml). The organic phase was separated, washed with water (3 × 100 ml), dried and evaporated to a foam. The crude product was triturated in ethyl acetate (10 ml). The white solid was collected by filtration, washed with ethyl acetate (2 × 5 ml) and dried *in vacuo* to give the title compound (684 mg, 54%). The ethyl acetate filtrate was concentrated and chromatographed on silica gel, eluting with diethyl ether to give an additional amount of the title compound (275 mg, 22%): MS (TSP+ve) *m*/*z* 439 [MH]⁺; NMR δ (DMSO-*d*₆) includes 7.24 (1H, d, *J* 10 Hz), 6.29 (1H, dd, J 10 and 2 Hz), 6.21 (1H, s), 5.72 and 5.54 (1H, 2m), 5.69 (1H, d, *J* 4 Hz), 4.2 (1H, m), 3.03 (1H, m), 1.50 (3H, s), 1.2 (3H, s), 0.96 (3H, d, *J* 7 Hz). (Found: C, 60.11; H, 5.45; S, 7.01. C₂₂H₂₄F₂O₅S requires C, 60.26; H, 5.52; S, 7.31%).

### Example 2

### 6α,9α-Difluoro-11β-hydroxy-16α-methyl-3-oxo-17α-propionyloxy-androsta-1,4-diene-17α-carbothioic acid S-(2-oxo-tetrahydro-furan-3-ylmethyl) ester

Powdered anhydrous potassium carbonate (69 mg, 0.5 mmol) was added to a stirred solution of 6α,9α-diffuoro-11β-hydroxy-16α-methyl-3-oxo-17α-propionyloxy-androsta-1,4-diene-17β-carbothioic acid (234 mg, 0.5 mmol) in dry DMF (2.5 ml). [6α,9α-Difluoro-11β-hydroxy-16α-methyl-3-oxo-17α-propionyloxy-androsta-1,4-diene-17β-carbothioic acid is a known material which may be prepared for example according to the method described in GB-A-2088877.] The mixture was stirred under nitrogen for 1 h and then treated with α-methylene-γ-butyrolactone (0.1 ml, 1.14 mmol) and the mixture was stirred for 20 h at room temperature. The solution was partitioned between water (25 ml) and ethyl acetate (25 ml). The organic phase was separated, washed with 2M HCl (20 ml), brine (2 × 25 ml), dried and evaporated to a solid. The crude product was purified by column chromatography on silica gel, and by preparative thin layer chromatography eluting with diethyl ether to give a mixture of the two diastereoisomers (37 mg) which were separated by HPLC (chiracel, 25 cm × 2 cm) eluting with 30% isopropanol-heptane at 6 ml/min and detecting at 240 nm to give the title compound isomer A (9 mg, 3%) MS (TSP+ve) *m*/*z* 567 [MH]⁺; NMR δ (CDCl₃) includes 7.15 (1H, d, *J* 10 Hz), 6.43 (1H, s), 6.38 (1H, d, *J* 10 Hz), 5.49 and 5.3 (1H, 2m), 4.4 (1H, m), 4.38 (1H, dt, *J* 7 and 2 Hz), 4.2 (1H, dt, *J* 10 and 7 Hz), 3.49 (1H, dd, *J* 14 and 5 Hz), 3.4 (1H, m), 3.10 (1H, dd, *J* 14 and 7.5 Hz), 2.95 (1H, m), 2.36 (2H, q. *J* 7.5 Hz), 1.53 (3H, s), 1.12 (3H, t, *J* 7.5 Hz), 1.05 (3H, s), 0.98 (3H, d, *J* 7 Hz). and the title compound isomer B (7 mg, 2%): MS (TSP+ve) *m*/*z* 567 [MH]⁺; NMR δ (CDCl₃) includes 7.15 (1H, d, *J* 10 Hz), 6.43 (1H, s), 6.39 (1H, d, *J* 10 Hz), 5.49 and 5.29 (1H, 2m), 4.4 (2H, dt, *J* 10 and 2 Hz), 4.20 (2H, dt, *J* 10 and 6 Hz), 3.40 (1H, dd, *J* 14 and 5 Hz), 3.3 (1H, m), 3.14 (1H, dd, *J* 14 and 8 Hz), 2.91 (1H, m), 2.35 (2H, q, *J* 7.5 Hz), 1.53 (3H, s), 1.12 (3H, t, *J* 7.5 Hz), 1.08 (3H, s), 1.01 (3H, d, *J* 7 Hz).

### Example 3

### Intermediate (i): (2-Oxo-tetrahydro-furan-4-ylsulfanyl)-acetic acid

Bromoacetic acid (588 mg, 4.33 mmol) was added to a stirred solution of β-mercapto-γ-butyrolactone (500 mg, 4.23 mmol) and triethylamine (0.59 ml, 4.23 mmol) in anhydrous tetrahydrofuran (10 ml) at 0°C under a nitrogen atmosphere. [β-Mercapto-γ-butyrolactone is a known material which may be prepared for example according to the method described by G. Fuchs, Ark. Kemi. 1968, 29, 379.] The reaction mixture was stirred for 15 minutes at 0°C and for 17 h at room temperature. Water (20 ml) and ethyl acetate (20 ml) were added and the organic phase was separated, washed with saturated brine and dried over anhydrous magnesium sulfate. Removal of the solvent under reduced pressure gave the title compound (437 mg, 59%): MS (TSP+ve) m/z 194 (M+NH₄)⁺; NMR δ (CDCl₃) includes 8.3-7.9 (1H, br), 4.65 (1H, dd, J 9.5 and 7 Hz), 4.21 (1H, dd, J 9.5 and 6 Hz), 3.88 (1H, m), 3.35 (2H, AB q, J 16 Hz), 2.98 (1H, dd, J 18 and 8 Hz), 2.56 (1H, dd, J 18 and 7 Hz).

### Intermediate (ii): (2-Oxo-tetrahydro-furan-4-ylsulfanyl)-acetyl chloride

Oxalyl chloride (0.316 ml, 3.62 mmol) was added dropwise to a stirred solution of (2-oxo-tetrahydro-furan-4-ylsulfanyl)-acetic acid (424 mg, 2.41 mmol) in anhydrous dichloromethane (5 ml) containing DMF (1 drop) under a nitrogen atmosphere. After 4 h the solvent was removed to give the title compound (434 mg, 93%): MS (TSP+ve) *m*/*z* 195 (M+H)+; NMR δ (CDCl₃) includes 4.63 (1H, dd, *J* 10 and 7 Hz), 4.19 (1H, dd, *J* 10 and 6 Hz), 3.81 (3H, m), 2.98 (1H, dd *J* 18 and 8 Hz), 2.52 (1H, dd, *J* 18 and 6.5 Hz).

### 6α,9α-Difluoro-11β-hydroxy-16α-methyl-3-oxo-17α-(2-oxo-tetrahydro-furan-4-ylsulfanyl-acetoxy)-androsta-1,4-diene-17β-carbothioic acid methyl ester

A solution of (2-oxo-tetrahydro-furan-4-ylsulfanyl)-acetyl chloride (354 mg, 1.82 mmol) in anhydrous dichloromethane (4 ml) was added, dropwise over 4 minutes to a stirred solution of 6α,9α-difluoro-11β,17α-dihydroxy-16α-methyl-3-oxo-androsta-1,4-diene-17β-carbothioic acid (300 mg, 0.73 mmol) [see Example 1] and triethylamine (0.254 ml, 1.82 mmol) in anhydrous dichloromethane (15 ml) at room temperature under a nitrogen atmosphere. The resulting solution was stirred for 1.5 h and then diethylamine (0.188 ml, 1.82 mmol) was added. After 1.5 h triethylamine (0.142ml, 1.02 mmol) was added followed by iodomethane (0.054 ml, 0.874 mmol) and the reaction mixture was stirred for a further 45 minutes. The reaction mixture was poured into water (30 ml) and extracted with dichloromethane (30 ml × 2). The combined organic extracts were washed with saturated brine (40 ml) and dried over anhydrous magnesium sulfate. Removal of the solvent under reduced pressure yielded a light brown foam which was chromatographed on silica gel using dichloromethane-ethyl acetate (3:1) as eluent. Removal of the solvent from the required fractions gave the title compound (317 mg; 72%): MS (ES+ve) *m*/*z* 585 (M+H)⁺; IR νₘₐₓ (KBr) 1780, 1742, 1686, 1666 cm⁻¹; NMR δ (CDCl₃) includes 7.12 (1H, d, *J* 10 Hz), 6.43 (1H, s), 6.38 (1H, d, *J* 10 Hz), 5.49 and 5.29 (1H, 2 m), 4.58 (1H, dd, *J* 9.5 and 7 Hz), 4.41 (1H, m), 4.16 (1H, m), 3.79 (1H, m), 3.39 (1H, m), 3.31 (2H, m), 2.91 (1H, dd *J* 18 and 8 Hz), 2.37 (3H, s), 1.52 (3H, s), 1.09 (3H, s), 1.05 (3H, d, *J* 7 Hz). (Found: C, 56.48; H, 5.83; S, 10.86.C₂₈H₃₄F₂O₇S₂.0.15CH₂Cl₂ requires C, 56.59; H, 5.79; S, 10.73%).

### Example 4

### Intermediate: 16α,17α-(2-Bromoethylidenedioxy)-6α,9α-difluoro-11β,21-dihydroxy-pregn-4-ene-3,20-dione

### Perchloric acid (70%, 1.62 ml, 18.8 mmol) was added to a stirred mixture of 21-acetyl-6α,9α-difluoro-11β,21-dihydroxy-16α,17α-

isopropylidenedioxy-pregn-4-ene-3,20-dione (2.33 g, 4.69 mmol), bromoacetaldehyde dimethylacetal (1.11 ml, 9.38 mmol) and sand (46.6 g) in heptane (58 ml) at room temperature. [21-Acetyl-6α,9α-difluoro-11β,21-dihydroxy-16α,17α-isopropylidenedioxy-pregn-4-ene-3,20-dione is a known material which may be prepared for example according to the method described in U.S. Patents Nos. 4524134, 4684610, 4704358 and 4749649 in the name of Upjohn.] After stirring for 17 h the heptane was removed by filtration and a further portion of heptane (60 ml) was added to the sand which was stirred for 5 minutes and then filtered. Ethyl acetate was added to the sand and the mixture stirred for 5 minutes and then filtered. This process was repeated three times using ethyl acetate (3 × 60 ml). The combined ethyl acetate filtrates were concentrated then passed through a silica gel plug eluting with ethyl acetate. The solvent was removed under reduced pressure and ethyl acetate (60 ml) and saturated sodium bicarbonate (60 ml) were added. The organic phase was separated, washed with water (60 ml) and saturated brine (60 ml) and dried over anhydrous magnesium sulfate. Removal of the solvent under reduced pressure yielded a brown foam which was chromatographed on silica gel using diethyl ether-ethanol (30:1) as eluent. Removal of the solvent from the required fractions gave the title compound (480 mg, 20%): MS (ES+ve) *m*/*z* 519 (M+H)⁺; NMR δ (CDCl₃) includes 6.14 (1H, s), 5.48 (1H, t, *J* 3Hz), 5.36 (1.5H. m), 5.18 (0.5H, m), 4.88 (1H, d, *J 20* Hz), 4.41 (1H, m), 4.22 (1H, d, *J* 20 Hz), 3.35 (2H, m), 1.51 (3H, s), 0.93 (3H, s).

### 6α,9α-Diftuoro-11β,21-dihydroxy-16α,17α-[2-(2-oxo-tetrahydro-furan-3-yl)sulfanyl]ethylidenedioxy-pregn-4-ene-3,20-dione

Triethylamine (0.315 ml, 2.26 mmol) was added to a stirred solution of 16α,17α-(2-bromoethylidene)dioxy-6α,9α-difluoro-11β,21-dihydroxy-pregn-4-ene-3,20-dione (470 mg, 0.905 mmol) and α-mercapto-γ-butyrolactone (267 mg, 2.26 mmol) in anhydrous DMF (3 ml) at room temperature under a nitrogen atmosphere. [α-Mercapto-γ-butyrolactone is a known material which may be prepared for example according to the method described by G. Fuchs, Ark. Kemi. 1968, 29, 379.] After stirring for 42 h ethyl acetate (30 ml) and water (30 ml) were added and the organic phase was separated, washed with saturated brine (30 ml) and dried over anhydrous magnesium sulfate. Removal of the solvent under reduced pressure yielded a brown oil which was chromatographed on silica gel using dichloromethane-ethyl acetate (1:1) as eluent. Removal of the solvent from the required fractions gave the title compound (185 mg, 37%): MS (ES+ve) *m*/*z* 557 (M+H)⁺; NMR δ (CDCl₃) includes 6.14 (1H, s), 5.57 (0.5H, t, *J* 3 Hz), 5.51 (0.5H, dd, *J* 5 and 3 Hz), 5.41-5.28 (1.5H, m), 5.18 (0.5H, m), 4.92-4.71 (1H, m), 4.47-4.16 (4H, m), 3.64 (1H, dd, *J* 8.5 and 4.5), 3.28 (0.5H, dd, *J* 14.5 and 2.5), 3.1-2.76 (2.5H, m), 1.52 (3H, s), 0.94 (3H, s). (Found: C, 56.93; H, 6.02; S, 5.35. C₂₇H₃₄F₂O₈S.0.2CH₂Cl₂ requires C, 56.96; H, 6.04; S, 5.59%).

### Example 5

### Intermediate (i): 21-Acetoxy-9α-fluoro-11β-hydroxy-3,20-dioxo-pregna-1,4-diene-16α-malonic acid diallyl ester

A solution of 21-acetoxy-9α-fluoro-11β-hydroxy-pregna-1,4,16-triene-3,20-dione (12.14 g, 30.16 mmol) in DMF (260 ml) was treated with diallyl malonate (6.8 g, 36.92 mmol) and DBU (5.11 g, 33.57 mmol). [21-Acetoxy-9α-fluoro-11β-hydroxy-pregna-1,4,16-triene-3,20-dione is a known material which may be prepared for example according to the method described by U. Ramesh, Tetrahedron Letters 1996, 37, 8403.] The reaction mixture was stirred for 11 days at room temperature, and then the solvent was removed under reduced pressure. The residue was dissolved in dichloromethane and washed with 1M HCl. The organic phase was separated, dried over magnesium sulfate and evaporated. The residue was chromatographed on silica gel, eluting with methanol-chloroform (3:97) to give the title compound (16.43 g, 93%) as a white solid: MS (FAB+ve) *m*/*z* 587 [MH]⁺; NMR δ (CDCl₃) includes 7.25 (1H, d, *J* 10 Hz), ), 6.33 (1H, dd, *J* 10 and 1.5 Hz), 6.11 (1H, s), 5.86 (2H, m), 5.27 (4H, m), 4.78 (1H, d, *J* 17 Hz), 4.60 (1H, d, *J* 6 Hz), 4.53 (1H, d, *J* 6 Hz), 4.43 (1H, d, *J* 17 Hz), 4.33 (1H, m), 3.33 (1H, m), 2.15 (3H, s), 1.55 (3H, s), 0.98 (3H, s).

### Intermediate (ii): 21-Acetoxy-9α-fluoro-11β-hydroxy-3,20-dioxo-pregna-1,4-diene-16α-acetic acid

A mixture of 21-acetoxy-16α-diallylmalonyl-9α-fluoro-11β-hydroxy-pregna-1,4-diene-3,20-dione (9.75 g, 16.6 mmol), palladium (II) acetate (74.6 mg, 0.33 mmol), triphenylphosphine (349 mg, 1.33 mmol), formic acid (1.91 g, 41.5 mmol) and triethylamine (5.55 g, 54.8 mmol) in dioxane (530 ml) was heated to reflux for 20 min. The reaction mixture was then concentrated under reduced pressure to a volume of approximately 250 ml and diluted with dichloromethane. The mixture was then acidified with 0.1M HCl, the organic phase was separated, dried over magnesium sulfate and evaporated. The residue was chromatographed on silica gel, eluting with methanol-chloroform (3:47) to give the title compound (4.02 g, 52%) as a white solid: MS (FAB-ve) m/z 461 [M-H]-; NMR δ (CDCl₃) includes 7.25 (1H, d, *J* 10 Hz), 6.23 (1H, dd, *J* 10 and 1 Hz), 6.01 (1H, s), 5.42 (1H, br), 4.71 (1H, d, *J* 17 Hz), 4.58 (1H, d, *J* 17 Hz), 4.13 (1H, m), 2.16 (3H, s), 1.55 (3H, s), 0.98 (3H, s).

### Intermediate (iii): 21-Acetoxy-9α-fluoro-11β,17α-dihydroxy-3,20-dioxo-pregna-1,4-diene-16α-acetic acid γ-lactone

A solution of 21-acetoxy-9α-fluoro-11β-hydroxy-3,20-dioxo-pregna-1,4-diene-16α-acetic acid (6.03 g, 13.04 mmol) in DMF (250 ml) was treated with CuCr₂O₄ (1 g, 33mol%), silica gel (500 mg), celite (500 mg) and acetic acid (25 drops). The reaction mixture was stirred vigorously and refluxed for a total of 5 h with additional quantities of CuCr₂O₄ (1 g), silica gel (500 mg), celite (500 mg) and acetic acid (25 drops) added at hourly intervals. The reaction mixture was then cooled to room temperature and the solvent removed under reduced pressure. The residue was chromatographed on silica gel, eluting with ethyl acetate-hexane (3:2) to give the title compound (2.55 g, 42%) as a white solid: MS (FAB+ve) *m*/*z* 461 [MH]⁺; NMR δ (CDCl₃) includes 7.24 (1H, d, *J* 10 Hz), 6.33 (1H, dd, *J* 10 and 2 Hz), 6.12 (1H, s), 4.88 (1H, d, *J* 18 Hz), 4.76 (1H, d, *J* 18 Hz), 4.41 (1H, m), 3.45 (1H, t, *J* 8 Hz), 2.75 (1H, dd, J 10 and 18 Hz), 2.63 (1H, m), 2.16 (3H, s), 1.55 (3H, s), 1.03 (3H, s).

### 9α-Fluoro-11β,17α,21-trihydroxy-3,20-dioxo-pregna-1,4-diene-16α-acetic acid γ-lactone

A suspension of 21-acetoxy-11β,17α-dihydroxy-9α-fluoro-3,20-dioxo-pregna-1,4-diene-16α-acetic acid γ-lactone (227 mg, 0.49 mmol) in DMSO (11 ml) and pH 7.2 phosphate buffer (95 ml) was treated with Tween 80 (30 drops) followed by esterase (EC 3.1.1.1; 14.5 mg/ml, 2.2 ml) at 37°C. The reaction mixture was stirred vigorously for 20 min and then cooled to 0°C and diluted with ethyl acetate. The aqueous phase was extracted several times with ethyl acetate, the combined ethyl acetate extracts were washed with brine, dried (MgSO₄) and the solvent evaporated. The residue was chromatographed on silica gel, eluting with ethyl acetate-hexane (4:1→9:1) to give the title compound (342.7 mg, 84%) as a white solid: MS (FAB+ve) m/z 419 [MH]⁺; NMR δ (CDCl₃) includes 7.17 (1H, d, *J* 10 Hz), 6.33 (1H, dd, *J* 10 and 2 Hz), 6.12 (1H, s), 4.58 (1H, d, *J* 20 Hz), 4.40 (1H, m), 4.28 (1H, d, *J* 20 Hz), 3.52 (1H, t, *J* 9 Hz), 2.77 (1H, dd, *J* 16 and 9 Hz), 2.65 (1H, m), 1.56 (3H, s), 1.01 (3H, s).

### Example 6

### Intermediate (i): 3-(3-lodopropylsulfanyl)-dihydro-2(3H)-furanone

α-Mercapto-γ-butyrolactone (772 mg, 6.53 mmol) was added to a stirred solution of 1,3-diiodopropane (1.12 ml, 9.75 mmol) and triethylamine (0.906 ml, 6.6 mmol) in anhydrous dichloromethane (6 ml) at 0°C under a nitrogen atmosphere. [α-Mercapto-γ-butyrolactone is a known material which may be prepared for example according to the method described by G. Fuchs, Ark. Kemi. 1968, 29, 379.] The reaction mixture was stirred for 30 minutes at 0°C and for 6 h at room temperature. The crude reaction mixture was adsorbed on silica gel and chromatographed, eluting with cyclohexane-ethyl acetate (3:1). Removal of the solvent from the required fractions under reduced pressure gave the title compound (920 mg, 49%): MS (TSP+ve) *m*/*z* 304 (M+NH₄)⁺; NMR δ (CDCl₃) includes 4.50-4.30 (2H, m), 3.51 (1H, dd, J 8 and 4 Hz), 3.30 (2H, t, J 6 Hz), 3.03-2.6 (3H, m), 2.22-2.04 (3H, m).

### 3-[3-[2-(4-Amino-3,5-dichlorophenyl)-2-hydroxyethylamino]propylsulfanyl]-dihydro-furan-2-one trifluoroacetate

Diisopropylethylamine (0.228 ml, 1.3 mmol) was added to a stirred solution of 2-amino-1-(4-amino-3,5-dichloro-phenyl)ethanol (504 mg, 2.28 mmol) in anhydrous DMF (4 ml), followed by a solution of 3-(3-iodopropylsulfanyl)-dihydro-2(3*H*)-furanone (340 mg, 1.19 mmol) in DMF (1 ml) at 20°C under a nitrogen atmosphere. [2-Amino-1-(4-amino-3,5-dichloro-phenyl)ethanol is a known material which may be prepared for example according to the method described by J. Keck, Arzneim. Forsch. 1972, 22, 861.] The reaction mixture was stirred for 20 h at room temperature and then the solvent was removed under reduced pressure. The crude reaction mixture was chromatographed on silica gel, eluting with methanol:chloroform:triethylamine (4:40:1). Removal of the solvent from the required fractions under reduced pressure gave an oil (408 mg) part of which was further purified by preparative HPLC (Spherisorb ODS-2, 25 x 2 cm) eluting with a gradient of acetonitrile-0.05% aqueous TFA at 15 ml/min collecting fractions with retention time of 10.4 min to give the title compound (103 mg, 17%): MS (TSP+ve) *m*/*z* 379 (M+H)⁺; NMR δ (CDCl₃) includes 7.29 (2H, s), 4.46-4.28 (2H, m), 3.73 (1H, dd, *J* 9 and 6 Hz), 3.35-3.05 (4H, m), 3.0-2.62 (3H, m) and 2.2-2.0 (3H, m).

### Example 7

### 6α,9α-Difluoro-11β-hydroxy-16α-methyl-3-oxo-17α-propionyloxy- androsta-1,4-diene-17β-carbothioic acid S-(2-oxo-1,3-dioxolan-4-yl) ester

A solution of 6α,9α-difluoro-11β-hydroxy-16α-methyl-3-oxo-17α-propionyloxy-3-oxo-androsta-1,4-diene-17β-carbothioic acid (5 g, 10.67 mmol) in DMF (50 ml) was treated with potassium carbonate (1.5 g, 10.85 mmol) and the resulting mixture was stirred under nitrogen for 1 h. After cooling in an ice-bath, chloroethylene carbonate (1.25 ml, 13.81 mmol) was added. The reaction mixture was stirred at room temperature for 20 h after which water (500 ml) and ethyl acetate (500 ml) were added. The organic phase was separated and washed with brine (150 ml), dried and evaporated to a foam. This was purified by column chromatography on silica gel, eluting with diethyl ether-cyclohexane (3:1) to give the title compound isomer A (878 mg, 15%); mp. 155-167 °C; MS (TSP+ve) 555 [MH]⁺; HRMS (ES+ve) found: 555.1873 [MH]⁺, C₂₇H₃₃F₂O₈S requires 555.1864; IR νₘₐₓ (KBr) 3351, 1820, 1741, 1670, 1635 cm⁻¹; NMR 8 (CDCl₃) includes 7.13 (1H, d, *J* 10 Hz), 6.44 (1H, s), 6.43-6.35 (2H, m), 5.49 and 5.30 (1H, 2m), 4.87 (1H, dd, *J* 9 and 8 Hz), 4.48 (1H, dd, *J* 9 and 5 Hz), 4.43 (1H, m), 3.36 (1H, m), 2.40 (2H, q, *J* 7 Hz), 1.54 (3H, s), 1.14 (3H, t; *J* 7 Hz), 1.11 (3H, s), 0.97 (3H, d, *J* 7 Hz); and the title compound isomer B (840 mg, 14%); mp. 234-236 °C; MS (TSP+ve) 555 [MH]⁺; HRMS (ES+ve) found: 555.1851 [MH]⁺, C₂₇H₃₃F₂O₈S requires 555.1864; IR νₘₐₓ (KBr) 3336, 1823, 1747, 1668, 1633 cm⁻¹; NMR δ (CDCl₃) includes 7.15 (1H, d, *J* 10 Hz), 6.44 (1H, s), 6.39 (1H, dd, *J* 10 and 2 Hz), 6.31 (1H, dd, *J* 8 and 5 Hz), 5.50 and 5.30 (1H, 2m), 4.86 (1H, dd, *J* 10 and 8 Hz), 4.53 (1H, dd, *J* 10 and 5 Hz), 4.42 (1H, m), 3.22 (1H, m), 2.38 (2H, q, *J* 7 Hz), 1.53 (3H, s), 1.16 (3H, s), 1.13 (3H, t, *J* 7 Hz), 1.02 (3H, d, *J* 7 Hz); (Found: C, 58.10; H, 5.91; S, 5.55. C₂₇H₃₂F₂O₈S requires C, 58.47; H, 5.82; S, 5.77 %).

### Example 8

### 6α,9α-Difluoro-11β-hydroxy-16α,17α-isopropylidenedioxy-3-oxo-androsta-1,4-diene-17β-carbothioic acid S-(2-oxo-1,3-dioxolan-4-yl) ester

A solution of 6α,9α-difluoro-11β-hydroxy-16α,17α-isopropytidenedioxy-3-oxo-androsta-1,4-diene-17β-carbothioic acid (200 mg, 0.44 mmol) in DMF (5 ml) was treated with potassium carbonate (61 mg, 0.44 mmol) and the resulting mixture was stirred under nitrogen for 0.5 h. After cooling in an ice-bath, chloroethylene carbonate (0.043 ml, 0.53 mmol) was added. The reaction mixture was stirred at room temperature for 2 h after which water (15 ml) and ethyl acetate (15 ml) were added. The organic phase was separated and washed with brine (15 ml), dried and evaporated to a solid. This was purified by column chromatography on silica gel, eluting with ethyl acetate-cyclohexane (1:1) to give the title compound isomer A (27 mg, 11%); mp. 247-250 °C; MS (TSP+ve) 541 [MH]⁺; IR νₘₐₓ (KBr) 3350, 1822, 1682, 1666, 1621 cm⁻¹; NMR δ (CDCl₃) includes 7.13 (1H, d, *J* 10 Hz), 6.45 (2H, m), 6.39 (1H, dd, *J* 10 and 2 Hz), 5.49 and 5.30 (1H, 2m), 4.99 (1H, d, *J* 5 Hz), 4.88 (1H, dd, *J* 9 and 8 Hz), 4.42 (2H, m), 3.50 (1H, br s), 1.54 (3H, s), 1.46 (3H, s), 1.24 (3H, s), 1.00 (3H, s), and the title compound isomer B (42 mg, 18%) mp. 254-257 °C; MS (TSP+ve) 541 [MH]⁺; IR νₘₐₓ (KBr) 3340, 1821, 1693, 1666, 1623 cm⁻¹; NMR δ (DMSO-*d*₆) includes 7.27 (1H, d, *J* 10 Hz), 6.40 (1H, dd, *J* 9 and 5 Hz), 6.32 (1H, d, *J* 10 Hz), 6.12 (1H, s), 5.75 and 5.55 (1H, 2m), 5.58 (1H, d, *J* 4 Hz), 4.93 (2H, m), 4.52 (1H, dd, *J* 9 and 6 Hz), 4.20 (1H, m), 1.50 (3H, s), 1.47 (3H, s), 1.21 (3H, s), 0.90 (3H, s); (Found: C, 57.5; H, 5.6; S, 5.8. C₂₆H₃₀F₂O₈S requires C, 57.8; H, 5.6; S, 5.9 %).

### Example 9

### 6α,9α-Difluoro-11β-hydroxy-16α-methyl-3-oxo-17α-propionyloxy-androsta-1,4-diene-17β-carboxylic acid O-(2-oxo-1,3-dioxolan-4-yl) ester

A solution of 6α,9α-difluoro-11β-hydroxy-16α-methyl-3-oxo-17α-propionyloxy-3-oxo-androsta-1,4-diene-17β-carboxylic acid (249 mg, 0.55 mmol) in DMF (5 ml) was treated with potassium carbonate (38 mg, 0.28 mmol) and the resulting mixture was stirred under nitrogen for 2 h. After cooling in an ice-bath, sodium iodide (20 mg, 0.13 mmol), was added followed by chloroethylene carbonate (0.456 ml, 5.52 mmol). The reaction mixture was stirred at room temperature for 20 h after which water (10 ml) and ethyl acetate (10 ml) were added. The organic phase was separated and washed with brine (10 ml), dried and evaporated to a foam. This was purified by column chromatography on silica gel, eluting with chloroform-ethanol (15:1) to give the title compound isomer A (23 mg, 8%); mp. 251-253 °C; MS (TSP+ve) 539 [MH]⁺; HRMS (ES+ve) found: 539.2068 [MH]⁺, C₂₇H₃₃F₂O₉ requires 539.2093; NMR δ (CDCl₃) includes 7.09 (1H, dd, *J* 10 and 1Hz), 6.77 (1H, dd, *J* 7 and 2 Hz), 6.44 (1H, s), 6.38 (1H, dd, *J* 10 and 2 Hz), 5.48 and 5.28 (1H, 2m), 4.61 (1H, dd, *J* 10 and 7 Hz), 4.39 (1H, m), 4.35 (1H, dd, *J* 10 and 2 Hz), 3.33 (1H, m), 2.39 (2H, q, *J* 7 Hz), 1.53 (3H, s), 1.14 (3H, t, *J* 7 Hz), 1.10 (3H, s), 0.94 (3H, d, *J* 7 Hz); and the title compound isomer B (14 mg, 5%); MS (TSP+ve) 539 [MH]⁺; HRMS (ES+ve) found: 539.2069 [MH]⁺, C₂₇H₃₃F₂O₉ requires 539.2093; NMR δ (CDCl₃) includes 7.10 (1H, dd, *J* 10 and 1 Hz), 6.62 (1H, dd, *J* 6 and 2 Hz), 6.44 (1H, s), 6.38 (1H, dd, *J* 10 and 2 Hz), 5.48 and 5.28 (1H, 2m), 4.65 (1H, dd, *J* 10 and 5 Hz), 4.55 (1H, dd, *J* 10 and 2 Hz), 4.41 (1H, m), 3.81 (1H, m), 3.24 (1H, m), 2.38 (2H, q, *J* 7 Hz), 1.53 (3H, s), 1.12 (6H, s and t, *J* 7 Hz), 0.96 (3H, d, *J* 7 Hz).

### Example 10

### 6α,9α-Difluoro-11β-hydroxy-16α-methyl-3-oxo-17α-propionyloxy-androsta-1,4-diene-17β-carbothioic acid S-(5-methyl-2-oxo-1,3-dioxol-4-ylmethyl) ester

A solution of 6α,9α-difiuoro-11β-hydroxy-16α-methyl-3-oxo-17α-propionyloxy-androsta-1,4-diene-17β-carbothioic acid (228 mg, 0.49 mmol) in DMF (4 ml) was treated with potassium carbonate (67 mg, 0.49 mmol) and the resulting mixture was stirred under nitrogen for 20 min at room temperature. After cooling in an ice-bath, a solution of 4-bromo-methyl-5-methyl-1,3-dioxol-2-one (123 mg, 0.64 mmol) in DMF (1 ml) was added. The reaction mixture was stirred at room temperature for 3 h after which the mixture was concentrated under reduced pressure. Water (25 ml) and ethyl acetate (25 ml) were added to the residue. The organic phase was separated and washed with brine (20 ml), dried and evaporated to a gum. This was purified by column chromatography on silica gel, eluting with chloroform-methanol (40:1). The appropriate fractions were combined and evaporated to dryness and the residue (115 mg) was triturated in diethyl ether (3 ml) to give the title compound (76 mg, 27%); MS (TSP+ve) 581 [MH]⁺; IR νₘₐₓ (KBr) 3412, 1821, 1740, 1668, 1629 cm⁻¹; NMR δ (CDCl₃) includes 7.11 (1H, dd, *J* 10 and 1 Hz), 6.44 (1H, s), 6.38 (1H, dd, *J* 10 and 2 Hz), 5.44 and 5.32 (1H, 2m), 4.41 (1H, m), 3.90 (2H, AB q, *J* 14 Hz), 3.34 (1H, m), 2.35 (2H, q, *J* 7 Hz), 2.17 (3H, s), 1.52 (3H, s), 1.12 (3H, t, *J* 7 Hz), 1.00 (3H, s), 0.97 (3H, d, *J* 7 Hz); (Found: C, 59.6; H, 6.2. C₂₉H₃₄F₂O₈S requires C, 60.0; H, 5.9%).

### Example 11

### 6α,9α-Difluoro-11β-hydroxy-16α,17α-isopropylidenedioxy-3-oxo-androsta-1,4-diene-17β-carbothioic acid S-(5-methyl-2-oxo-1,3-dioxol-4-ylmethyl) ester

A solution of 6α,9α-difluoro-11β-hydroxy-16α,17α-isopropylidenedioxy-3-oxo-androsta-1,4-diene-17β-carbothioic acid (200 mg, 0.44 mmol) in DMF (5 ml) was treated with potassium carbonate (61 mg, 0.44 mmol) and the resulting mixture was stirred under nitrogen for 0.5 h at room temperature. After cooling in an ice-bath, 4-bromomethyl-5-methyl-1,3-dioxol-2-one (102 mg, 0.53 mmol) was added. The reaction mixture was stirred at room temperature for 22 h after which water (20 ml) and ethyl acetate (20 ml) were added. The organic phase was separated and washed with brine (20 ml), dried and evaporated to a solid. This was purified by column chromatography on silica gel, eluting with ethyl acetate-cyclohexane (1:1) to give the title compound (212 mg, 85%); mp. 241-244 °C; MS (TSP+ve) 567 [MH]⁺; IR νₘₐₓ (KBr) 3418, 1822, 1667, 1663 cm⁻¹;NMR δ (CDCl₃) includes 7.12 (1H, d, *J* 10 Hz), 6.44 (1H, s), 6.38 (1H, dd, *J* 10 and 2 Hz), 5.49 and 5.29 (1H, 2m), 4.98 (1H, d, *J* 5 Hz), 4.40 (1H, m), 4.03 (1H, d, *J* 18 Hz), 3.70 (1H, d, *J* 18 Hz), 2.19 (3H, s), 1.53 (3H, s), 1.44 (3H, s), 1.22 (3H, s), 0.87 (3H, s); (Found: C, 59.4; H, 5.7; S, 5.6. C₂₈H₃₂F₂O₈S requires C, 59.4; H, 5.7; S, 5.7%).

### Example 12

### 6α,9α-Difluoro-11β-hydroxy-16α-methyl-3-oxo-17α-propionyloxy-androsta-1,4-diene-17β-carboxylic acid O-(5-methyl-2-oxo-1,3-dioxol-4-ylmethyl) ester

A solution of 6α,9α-difluoro-11β-hydroxy-16α-methyl-3-oxo-17α-propionyloxy-androsta-1,4-diene-17β-carboxylic acid (220 mg, 0.49 mmol) in DMF (4 ml) was treated with potassium carbonate (67 mg, 0.49 mmol) and the resulting mixture was stirred under nitrogen for 20 min at room temperature. After cooling in an ice-bath, a solution of 4-bromomethyl-5-methyl-1,3-dioxol-2-one (123 mg, 0.64 mmol) in DMF (1 ml) was added. The reaction mixture was stirred at room temperature for 3.5 h after which the mixture was concentrated under reduced pressure. Water (30 ml) and ethyl acetate (30 ml) were added to the residue. The organic phase was separated and washed with brine (25 ml), dried and evaporated to a foam. This was purified by column chromatography on silica gel, eluting with chloroform-methanol (60:1). The appropriate fractions were combined and evaporated to dryness and the residue (65 mg) was triturated in diethyl ether (3 ml) to give the title compound (26 mg, 9%); MS (TSP+ve) 565 [MH]⁺; IR νₘₐₓ (KBr) 3393, 1822, 1747, 1669, 1630 cm⁻¹; NMR δ (CDCl₃) includes 7.11 (1H, d, *J* 10 Hz), 6.43 (1H, s), 6.38 (1H, dd, *J* 10 and 2 Hz), 5.44 and 5.32 (1H, 2m), 4.95 and 4.80 (1H each, d, *J* 14 Hz), 4.39 (1H, m), 3.28 (1H, m), 2.35 (2H, q, *J* 7 Hz), 2.20 (3H, s), 1.52 (3H, s), 1.15 (3H, t, *J* 7 Hz), 0.98 (3H, s), 0.93 (3H, d, *J* 7 Hz); (Found: C, 60.7; H, 6.35. C₂₉H₃₄F₂O₉0.5H₂O requires C, 60.7; H, 6.15%).

### Pharmacological Activity

### (A). Glucocorticoid activity

The pharmacological activity was studied in a functional *in vitro* assay to demonstrate glucocorticoid activity which is generally predictive of anti-inflammatory or anti-allergic activity *in vivo*.

The functional assay used was a modification of the method described by T.S Berger et al, of J. of Steroid Biochem. Molec. Biol. 1992, 41 (3-8), 733-738, "Interaction of Glucocorticoid analogues with the Human Glucocorticoid Receptor".

Thus, Hela cells were stably transfected with a detectable reporter gene (secreted placental alkaline phosphatase, sPAP) under the control of a glucocorticoid response promoter (the LTR of the mouse mammary tumour virus, MMTV).

Various concentrations of standard (dexamethasone) or compounds of the invention were incubated with transfected Hela cells for 72 hours. At the end of the incubation, substrate (p-nitrophenol acetate) for sPAP was added and the product measured by a spectrophotometric method. Increased absorbance reflected increased sPAP transcription and concentration-response lines were constructed such that EC₅₀-values could be estimated.

In this test, the compounds of Examples 1, 2, 3, 4 and 5 had EG₅₀-values of less than about 250nM. The compounds of Example 7 (isomers A and B) and 10 had EC₅₀-values of less than 500nM.

### (B). β₂-Agonist activity

The compound of Example 6 was tested for its ability to cause relaxation of electrically induced contractile responses in guinea pig tracheal strips as described by Coleman and Nials, Journal of Pharmacological Methods 1989, 21, 71-86. EC₅₀-values were obtained for the test compound and for the standard isoprenaline for the same tracheal strip The EC₅₀-value for the compound of Example 6 was found to be 5.3 times greater than that of the isoprenaline standard.

### Stability in Human Plasma

The stability of various of the compounds of the Examples in human plasma was tested using the following 'stability in human plasma' test method: 500µl aliquots of human plasma in screw capped polypropylene tubes were preincubated at 37°C in a waterbath for 5 minutes. The plasma samples were then spiked with 5µl of test compound (nominally 5mg/ml in DMSO). Aliquots (100µl) were removed immediately and after 5 minutes and mixed with an equal volume of acetonitrile. The samples were centrifuged and the supernatants were transferred to autosampler vials for HPLC and half-life analysis.

In the HPLC procedure, aliquots (20µl) of all the supernatants were injected onto a Zorbax Rx C8 column (250 x 4.6mm; Hichrom). The column was maintained at 40°C and eluted at a flow rate of 1.0 mL/min with a mobile phase of acetonitrile: 50 mM ammonium formate (65:35) adjusted to pH 4.2 with formic acid. Detection was by UV absorbance at 240nm, and chromatographic peak areas for both parent and metabolite were measured

To determine the half-life, for each compound peak areas were plotted against time on a log-linear scale, and the half lives determined by extrapolation or interpolation of a straight line joining two points.

All the isomer/compounds of the Examples were found to be unstable in human plasma indicating that they are expected to possess an advantageous *in vivo* side effect profile. The compounds/isomers of all the Examples show half-lives of less than 1 h. The compounds of Examples 3, 4 and 6 to 12 show half-lives of less than 10 min.

### Hydrolysis by human serum paraoxonase

The susceptibility to hydrolysis by human serum paraoxonase of certain of the compounds of the Examples was assessed using the test protocol of the 'enzymatic hydrolysis test method' described herein.

The compounds of Examples 3, 4, 7 (isomer A), 9 (isomer A) and 12 were all found to have been hydrolysed by the paraoxonase enzyme. Similar results were obtained for the compounds of Examples 2, 3, 4, 6 and 7 (isomer B) when the test was repeated using purified lactonase enzyme obtained from human plasma.

### Stability in human lung S9

The stability of the compound of Examples 4 and 6 in a model human lung environment was assessed using the following test protocol: Incubations were carried out, at 37°C, in 500µL volumes of human lung S9 diluted 1:1 with 10mM phosphate buffer pH 7.4 to which was added 50µl of 30mg/ml (Aq) NADPH. The reactions were started by the addition of 5µl of test compound (nominally 5mg/ml solution in DMSO). Aliquots (100µl) were removed immediately and after 1 hour and mixed with an equal volume of acetonitrile to stop the reaction. Samples were centrifuged and the supernatants were transferred to autosampler vials for HPLC analysis and half-life analysis using methods identical to those described in the 'stability in human plasma' test protocol described above. Control incubations containing no lung S9 were also included.

The compounds of Examples 4 and 6 were only slowly hydrolysed in this lung S9 preparation (half-life >60min). It is thus illustrated that these compounds show stability in a target tissue environment (in this case the lung) whilst being rapidly inactivated in a plasma environment.

## Claims

1. A method of identifying a compound capable of providing a therapeutic effect at a target site within a human or animal body with reduced systemic potency to said body comprising:
(a) Comparing the susceptibility to hydrolysis of said compound in the presence of a purified lactonase enzyme or a recombinant form thereof to the corresponding susceptibility in the absence of said lactonase enzyme; and
(b) Selecting a compound on the basis of enhanced susceptibility to hydrolysis in the presence of said lactonase enzyme.

2. Method according to claim 1, wherein said compound is an anti-inflammatory or anti-allergic compound.

3. Method according to claim or claim 2, wherein said compound is a glucocorticosteroid.

4. Method according to any of daims 1 to 3, wherein said compound is capable of providing a therapeutic effect in the lung, gastrointestinal tract, skin, eyes and joints.

5. Method according to any of claims 1 to 4, the half-life of said compound in the presence of lactonase enzyme is less than 30 minutes, preferably less than. 10 minutes.

## Revendications

1. Procédé d'identification d'un composé capable de fournir un effet thérapeutique à un site cible dans un organisme humain ou animal, ayant une activité systémique réduite pour ledit organisme, comprenant :
(a) La comparaison de la susceptibilité à l'hydrolyse dudit composé en présence d'une enzyme lactonase purifiée ou d'une forme recombinée de celle-ci à la susceptibilité correspondante en l'absence de ladite enzyme lactonase, et
(b) La sélection d'un composé sur la base de la susceptibilité accrue à l'hydrolyse en présence de ladite enzyme lactonase.

2. Procédé selon la revendication 1, dans lequel ledit composé est un composé anti-inflammatoire ou antiallergique.

3. Procédé selon la revendication 1 ou 2, dans lequel ledit composé est un glucocorticostéroïde.

4. Procédé selon l'une quelconque des revendications 1 à 3, dans lequel ledit composé est capable de fournir un effet thérapeutique dans le poumon, le tractus gastro-intestinal, la peau, les yeux et les articulations.

5. Procédé selon l'une quelconque des revendications 1 à 4, dans lequel la demi-vie dudit composant en présence de l'enzyme lactonase est inférieure à 30 minutes, de préférence inférieure à 10 minutes.

## Patentansprüche

1. Verfahren zur Identifizierung einer Verbindung, die eine therapeutische Wirkung an einem Zielort innerhalb eines menschlichen oder tierischen Körpers mit reduzierter systemischer Wirksamkeit auf den Körper bereitstellen kann, umfassend:
(a) Vergleichen der Anfälligkeit der Verbindung für die Hydrolyse in Gegenwart eines gereinigten Lactonase-Enzyms oder einer rekombinanten Form davon mit der entsprechenden Anfälligkeit in Abwesenheit des Lactonase-Enzyms; und
(b) Auswählen einer Verbindung auf der Basis gesteigerter Anfälligkeit für die Hydrolyse in Gegenwart des Lactonase-Enzyms.

2. Verfahren gemäss Anspruch 1, worin die Verbindung eine entzündungshemmende oder antiallergische Verbindung ist.

3. Verfahren gemäss Anspruch 1 oder 2, worin die Verbindung ein Glucokortikosteroid ist.

4. Verfahren gemäss einem der Ansprüche 1 bis 3, worin die Verbindung eine therapeutische Wirkung in der Lunge, im Magen-Darm-Trakt, in der Haut, in den Augen und in den Gelenken bereitstellen kann.

5. Verfahren gemäss einem der Ansprüche 1 bis 4, wobei die Halbwertszeit der Verbindung in Gegenwart von Lactonase-Enzym weniger als 30 Minuten beträgt, bevorzugt weniger als 10 Minuten.
